# FASCICULE DE BREVET EUROPEEN

(11) **EP 3 318 141 B1**
(45) Date de publication et mention de la délivrance du brevet: **01.01.2020**
(21) Numéro de dépôt: 17200427.7
(22) Date de dépôt: 07.11.2017
(51) Int. Cl.: A41B 11/12, A61F 13/08, D04B 1/16

(54) **ARTICLE TEXTILE SOUS FORME D'UNE BANDE AEREE OBTENUE PAR TRICOTAGE OU TISSAGE, ET APTE A CONSTITUER LA PARTIE SUPERIEURE D'UN BAS OU D'UNE CHAUSSETTE**
TEXTILARTIKEL IN FORM EINES LUFTDURCHLÄSSIGEN TEXTILBANDES, DAS DURCH WIRKEN ODER WEBEN HERGESTELLT WIRD UND DAFÜR GEEIGNET IST, DEN OBEREN TEIL EINES STRUMPFS ODER EINER SOCKE ZU BILDEN
TEXTILE ITEM IN THE FORM OF A LOOSE-WOVEN STRIP OBTAINED BY WEAVING OR KNITTING, AND CAPABLE OF FORMING THE UPPER PART OF A STOCKING OR SOCK

(30) Priorité: 07.11.2016 FR 1660757
(43) Date de publication de la demande: 09.05.2018
(73) Titulaire: Fontanille Scop, 43000 Espaly-Saint-Marcel (FR)
(72) Inventeur: DE GOLBERY, Eric, 43370 CUSSAC SUR LOIRE (FR); RIVET, Cyril, 43000 LE PUY EN VELAY (FR); GARNIER, Didier, 43000 ESPALY SAINT-MARCEL (FR)
(74) Mandataire: Cabinet Laurent & Charras

(56) Documents cités:
- EP-A1- 2 886 691
- FR-A1- 3 034 311
- US-A1- 2016 120 233
- US-B1- 7 748 240

## Description

L'invention se rattache au secteur technique des articles textiles, et concerne plus particulièrement une bande obtenue par tricotage ou tissage.

L'invention trouve une application avantageuse dans le domaine des produits de contention, tels que les bas ou chaussettes, et plus généralement tout type d'articles vestimentaires, avec ou non des capacités de contention.

Différentes solutions techniques ont été proposées pour équiper la partie supérieure des bas ou des chaussettes d'une bande, qui adhère par élasticité à la peau.

On peut citer, par exemple, l'enseignement du brevet FR 1 540 295, qui divulgue une bande élastique de fixation revêtue sur l'une de ses faces, au moins, d'un élastomère de silicone, dont les caractéristiques évitent tous phénomènes de glissement, sans pour autant nuire à la fonction d'élasticité de la bande, en considérant le caractère extensible du silicone.

Le brevet FR 2 930 708 décrit une bande de fixation d'un article textile présentant une enduction de silicone, ayant des caractéristiques d'adhésivité immédiate, de viscoélasticité, avec un taux de transmission de vapeur d'eau et une biocompatibilité.

Généralement, le silicone est appliqué par un procédé d'enduction, sous forme d'un film, sur l'une des faces de l'article textile. Il en résulte que des phénomènes de macération peuvent apparaître, au niveau de la zone enduite de silicone, pour obtenir l'effet antiglisse. En effet, même si la bande obtenue par tissage ou tricotage, présente une structure textile suffisamment aérée, ce n'est pas le cas de la zone siliconée, qui constitue une zone imperméable ne permettant pas l'évacuation de la sueur. Des risques d'irritation peuvent donc apparaître.

Pour tenter de remédier à ces inconvénients, une solution avantageuse ressort de l'enseignement du brevet FR 3 006 626, dont le Demandeur de la présente est également titulaire. Ce brevet concerne un complexe textile présentant sur au moins l'une de ses faces, une zone revêtue d'une couche de silicone, qui reçoit un film protecteur. Des micros-perforations sont réalisées à la fois dans l'épaisseur du film protecteur, et dans la zone revêtue de la couche de silicone. L'article textile présente des caractéristiques d'allongement pour être étirable. Il ressort de ces caractéristiques, qu'une aération de la partie siliconée du support textile permet l'évacuation de la sueur.

Cette solution nécessite toutefois, une installation spéciale pour la dépose par enduction ou autrement, de la couche de silicone sur une partie ou la totalité de la bande textile. De même, cette couche de silicone peut nuire à l'effet esthétique de l'article textile considéré.

Le brevet FR 3 034 311 décrit un article textile selon le préambule des revendications 1 et 2. L'article comprend des fils adhérents aptes à adhérer à la peau, comprenant un fil d'âme recouvert d'une gaine en matériau adhérent, par exemple en silicone. Le titre des fils adhérents est compris entre 100 et 600 dtx. La surface de contact avec la peau est donc faible.

L'invention s'est fixée pour but de remédier à ces inconvénients d'une manière simple, sûre, efficace et rationnelle.

Le problème que se propose de résoudre l'invention, est de réaliser un article textile sous forme d'une bande aérée obtenue par tricotage ou tissage, ayant des qualités d'adhérence sur la peau d'un utilisateur, sans pour autant nuire à l'aération de la bande, autrement dit, en supprimant la couche de silicone.

Pour résoudre un tel problème, l'article textile sous forme d'une bande aérée obtenue par tricotage ou tissage, et apte à constituer la partie supérieure d'un bas ou d'une chaussette, présente sur au moins une face, des flottés de chaîne ou de trame en fils silicone ou en fils enduits de silicone en combinaison avec une armure apte à conférer à ladite bande une capacité d'allongement pour être étirable en chaîne et un blocage en trame.

Selon un premier mode de réalisation défini à la revendication 1, mettant en œuvre des flottés de chaîne, l'article est caractérisé en ce que le titre du fil silicone ou fil enduit de silicone formant les flottés de chaîne est compris entre 800 et 3500 dtex environ ; et en ce que dans le cas d'un tricotage, les flottés de chaîne sont parallèles aux chaînettes qui incorporent une âme ayant des capacités d'allongement, la trame étant réalisée en fil rigide, non extensible lorsqu'il est soumis à une traction manuelle, dans les conditions normales de température et de pression (CNTP) ; et dans le cas d'un tissage, les flottés de chaîne sont perpendiculaires aux fils de trame, qui sont rigides, non extensibles lorsqu'ils sont soumis à une traction manuelle, dans les conditions normales de température et de pression (CNTP), les fils de chaîne ayant une capacité d'allongement.

Selon un deuxième mode de réalisation défini à la revendication 2, mettant en œuvre des flottés de trame, l'article est caractérisé en ce que le titre du fil silicone ou fil enduit de silicone formant les flottés de trame est compris entre 800 et 3500 dtex environ ; et en ce que dans le cas d'un tricotage, les flottés de trame sont perpendiculaires aux chaînettes qui incorporent une âme ayant des capacités d'allongement ; et dans le cas d'un tissage, les flottés de trame sont perpendiculaires aux fils de chaîne ayant une capacité d'allongement.

Il résulte de ces caractéristiques que les fils silicone ou enduits de silicone sont mélangés avec les autres fils, qui constituent la bande. Les caractéristiques résultant du fil silicone, sont réparties de manière homogène sur la largeur de la bande. La bande ainsi obtenue est perméable, évitant, par conséquent, tous phénomènes de macération, et améliorant d'une manière significative le confort de l'utilisateur. De plus, la suppression du film silicone permet d'utiliser une soudure à ultrasons, pour la fixation de la bande, par exemple à la partie supérieure d'une chaussette ou d'un bas, améliorant le confort. Enfin, le titre des fils silicone ou enduits de silicone impacte la surface de contact avec la peau, et donc l'adhérence de l'article.

Le flotté de fils silicone ou des fils enduits de silicone est adapté en fonction du dessin et du rapport d'armure de l'article textile.

Un fil rigide est non extensible lorsqu'il est soumis à une traction manuelle, dans les conditions normales de température et de pression (CNTP).

De préférence, le titre du fil silicone ou enduit de silicone est compris entre 1500 et 2500 dtex, par exemple égal à 2000 dtex.

Plus le titre est important, plus la surface de contact avec la peau est importante, plus l'adhérence est bonne.

Les fils silicones ou enduits de silicone peuvent être de sections ronde ou ovale, être translucides ou mates.

L'invention est exposée ci-après plus en détail à l'aide des figures des dessins annexés dans lesquels :
- la figure 1 est un exemple d'armure de la bande textile obtenue par tricotage à mailles jetées, dans le cas d'un flotté de chaîne, des fils silicones ou des fils enduits de silicone.
- la figure 2 est un exemple d'armure de la bande obtenue par tissage, dans le cas d'un flotté de chaîne, des fils silicones, ou des fils enduits de silicone.
- la figure 3 est un exemple d'armure de la bande obtenue par tricotage dans le cas d'un flotté de trame, des fils silicones, ou des fils enduits de silicone.
- la figure 4 est un exemple d'armure de la bande obtenue par tissage dans le cas d'un flotté de trame, des fils silicones ou des fils enduits de silicone.
- La figure 5 est un exemple de réalisation d'une bande extensible avec incorporation de fils silicones, selon l'invention.

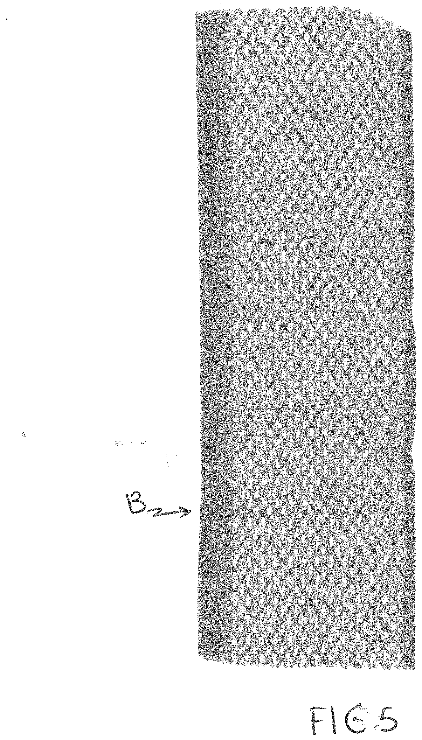

On a illustré figure 5, une bande (B), présentant des capacités d'allongement pour être étirée et incorporant des fils silicones. La bande peut être obtenue par tous moyens connus et appropriés, par tissage ou tricotage, et présente une structure aérée. Plus particulièrement, la bande est obtenue à partir de tout type de fils, en polyamide, en polyester..., pour obtenir une extension en chaîne avec, par exemple, incorporation de fils élasthanne, et un blocage en trame.

Selon l'invention, la bande (B), obtenue par tricotage ou tissage, présente sur au moins une face, des flottés (la), de chaîne ou de trame, en fils (1) réalisés en silicone, ou enduits de silicone, en combinaison avec l'armure de la bande, afin de lui conférer, comme indiqué, une capacité d'allongement pour être étirable en chaîne, et un blocage en trame.

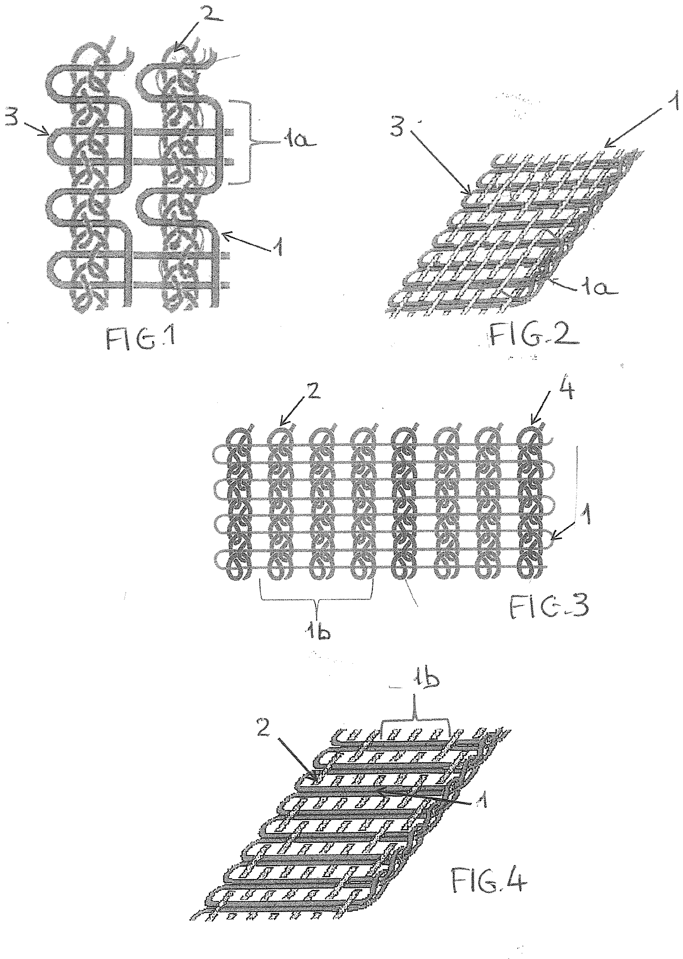

Les figures 1 et 2 montrent un flotté de chaîne (la) du fil silicone (1), ou du fil enduit de silicone dans le cas d'une armure obtenue par tricotage, figure 1, et dans le cas d'une armure obtenue par tissage, figure 2.

A la figure 1, les chaînettes sont désignées par (2), et réalisées en tous types de fils rigides, avec incorporation d'une âme en élasthanne, par exemple.

Les trames sont désignées par (3), et réalisées par tous types de fils rigides. Ainsi, son armure confère à la bande une capacité d'allongement en chaîne (chainettes (2) réalisées avec des fils rigides et une âme élastique) et un blocage en trame (fils de trame (3) rigides entrecroisés avec les chainettes (2), ce qui limite le déplacement transversal des chainettes (2)).

On voit que les flottés de chaîne (la) sont parallèles aux chaînettes (2). Les fils silicones (1), ou les fils enduits de silicone, sont donc tricotés avec les chaînettes (2), en réalisant des flottés de chaîne (la), qui relient au moins deux mailles. Ce sont ces flottés de chaîne, qui améliorent l'adhérence de la bande avec la peau de l'utilisateur.

La figure 2 correspond à une bande, selon les caractéristiques de l'invention, dont l'armure est obtenue par tissage. Les fils de trame sont toujours désignés par (3), tandis que les chaînes présentent les flottés de chaîne (la), des fils silicones (1) ou des fils enduits de silicone.

Les figures 3 et 4 correspondent à une bande, dont l'armure est réalisée par tricotage, figure 3, ou par tissage, figure 4, dont les flottés (1b) des fils silicone (1), ou des fils enduits de silicone sont disposés, non plus en chaîne, mais en trame.

Lorsque la bande est obtenue par tricotage, elle comprend des chaînettes (2), mais également des chaînettes rebrodées (4), afin de lier les fils silicones, ou les fils enduits de silicone. Ces chaînettes (4) sont faites dans un deuxième temps, en repassant la bande dans le métier avec une aiguille perforante, afin de lier les fils silicones (1), ou enduits silicone, en trame. Les flottés de trame (1b) sont perpendiculaires à la chaînette.

A la figure 4, qui correspond à une bande obtenue par tissage, la trame est réalisée par les fils silicones (1), ou les fils enduits de silicone, en réalisant des flottés de trame (1b), par rapport aux fils de chaîne (5).

Le but recherché est donc de réaliser une armure avec un maximum de flottés de chaîne (la), ou de trame (1b), de fils silicones (1) ou de fils enduits de silicone sur une face de la bande, obtenue par tissage ou tricotage, afin de faire apparaître en surface, sur une certaine distance, lesdits fils silicones, ou enduits de silicone, pour obtenir la propriété d'adhérence recherchée de l'article textile. Les fils silicones, ou les fils enduits de silicone, sont répartis sur la surface de la bande, en laissant des parties non siliconées de fils, afin de ne pas altérer la respirabilité de la bande. Les fils silicones (1), ou les fils enduits de silicone sont introduits sur le métier après ourdissage, sur une ensouple et adaptation nécessaire de certains paramètres, notamment au niveau de la tension exercée entre l'ensouple et le cantre, la vitesse, l'espacement entre les fils et le trajet, du fil simplifié du cantre vers l'ensouple.

Les fils de silicone (1) ou enduits de silicone sont de sections ronde ou ovale, en étant mates ou translucides.

À titre d'exemple indicatif, la bande tissée ou tricotée peut-être obtenue à partir de fils en polyamide de 40 à 220 dtex, de fils élasthannes de 280 à 570 dtex, de polyester ou de polyamide mono-filament, de l'ordre de 77 dtex et de fils silicone de l'ordre de 800 à 3500 dtex.

Les avantages ressortent bien de la description.

## Revendications

1. Article textile sous forme d'une bande aérée obtenue par tricotage ou tissage, et apte à constituer la partie supérieure d'un bas ou d'une chaussette, l'article présentant sur au moins une face des flottés de chaîne (la) en fils silicone (1) ou en fils enduits de silicone, en combinaison avec une armure apte à conférer à ladite bande une capacité d'allongement pour être étirable en chaîne et un blocage en trame, ***caractérisé* en ce que** le titre du fil silicone (1) ou fil enduit de silicone formant les flottés de chaîne (la) est compris entre 800 et 3500 dtex environ ;
et **en ce que** :
- dans le cas d'un tricotage, les flottés de chaîne (la) sont parallèles aux chaînettes (2) qui incorporent une âme ayant des capacités d'allongement, la trame étant réalisée en fil rigide (3), non extensible lorsqu'il est soumis à une traction manuelle, dans les conditions normales de température et de pression (CNTP) ; et
- dans le cas d'un tissage, les flottés de chaîne (la) sont perpendiculaires aux fils (3) de trame, qui sont rigides, non extensibles lorsqu'ils sont soumis à une traction manuelle, dans les conditions normales de température et de pression (CNTP), et les fils de chaîne ayant une capacité d'allongement.

2. Article textile sous forme d'une bande aérée obtenue par tricotage ou tissage, et apte à constituer la partie supérieure d'un bas ou d'une chaussette, l'article présentant sur au moins une face des flottés de trame (1b) en fils silicone (1) ou en fils enduits de silicone, en combinaison avec une armure apte à conférer à ladite bande une capacité d'allongement pour être étirable en chaîne et un blocage en trame, ***caractérisé* en ce que** le titre du fil silicone (1) ou fil enduit de silicone formant les flottés de chaîne (la) est compris entre 800 et 3500 dtex environ ;
et **en ce que** :
- dans le cas d'un tricotage, les flottés de trame (1b) sont perpendiculaires aux chaînettes (2) qui incorporent une âme ayant des capacités d'allongement ; et
- dans le cas d'un tissage, les flottés de trame (1b) sont perpendiculaires aux fils de chaîne ayant une capacité d'allongement.

3. Article textile selon l'une quelconque des revendications 1 ou 2, ***caractérisé* en ce que** le titre du fil silicone (1) ou fil enduit de silicone est compris entre 1500 et 2500 dtex.

4. Article textile selon l'une quelconque des revendications 1 ou 2, ***caractérisé* en ce que** le fil silicone (1) ou fil enduit de silicone est de section ronde.

5. Article textile selon l'une quelconque des revendications 1 ou 2, ***caractérisé* en ce que** le fil silicone (1) ou fil enduit de silicone est de section ovale.

6. Article textile selon l'une quelconque des revendications 1 ou 2, ***caractérisé* en ce que** le fil silicone (1) ou fil enduit de silicone est translucide.

7. Article textile selon l'une quelconque des revendications 1 ou 2, ***caractérisé* en ce que** le fil silicone (1) ou fil enduit de silicone est mate.

## Patentansprüche

1. Textiler Artikel in Form eines lockeren Bandes, das durch Stricken oder Weben hergestellt wird, der geeignet ist, den oberen Teil eines Strumpfs oder Wadenstrumpfs zu bilden, wobei der Artikel mindestens auf einer Seite Kettenflottierungen (1a) aus Silikonfäden (1) oder mit silikongetränkten Fäden in Kombination mit einer Bewehrung aufweist, durch die dieses Band eine Dehnungsfähigkeit erhält, um in der Kette dehnbar zu sein und im Schuss blockiert zu werden, ***dadurch gekennzeichnet, dass*** der Titer des Silikonfadens (1) oder des silikongetränkten Fadens, der die Kettenflottierungen (1a) bildet, zwischen ungefähr 800 und 3.500 dtex liegt; und dadurch, dass:
- wenn es sich um ein Gestrick handelt, die Kettenflottierungen (1a) parallel zu den Ketten (2) sind, die einen Kern enthalten, der über Dehnungsfähigkeiten verfügt, wobei der Schuss mit einem starren Faden (3) ausgeführt wird, der nicht dehnbar ist, wenn er einem manuellen Zug unter normalen Temperatur- und Druckbedingungen (CNTP) ausgesetzt wird; und
- im Falle eines Gewebes, die Kettenflottierungen (1a) senkrecht zu den Schussfäden (3) sind, die starr und nicht dehnbar sind, wenn sie einem manuellen Zug unter normalen Temperatur- und Druckbedingungen (CNTP) ausgesetzt sind; und wobei die Kettfäden über eine Dehnungsfähigkeit verfügen.

2. Textiler Artikel in Form eines lockeren Bandes, das durch Stricken oder Weben hergestellt wird, der geeignet ist, den oberen Teil eines Strumpfs oder Wadenstrumpfs zu bilden, wobei der Artikel mindestens auf einer Seite Schussflottierungen (1b) aus Silikonfäden (1) oder silikongetränkten Fäden in Kombination mit einer Bewehrung aufweist, durch die dieses Band eine Dehnungsfähigkeit erhält, um in der Kette dehnbar zu sein und im Schuss blockiert zu werden, ***dadurch gekennzeichnet, dass*** der Titer des Silikonfadens (1) oder des silikongetränkten Fadens, die die Kettenflottierungen (1a) bilden, zwischen ungefähr 800 und 3.500 dtex liegt; und dadurch, dass:
- im Falle eines Gestricks die Schussflottierungen (1b) senkrecht zu den Ketten (2) liegen, die einen dehnungsfähigen Kern enthalten; und
- im Falle eines Gewebes die Schussflottierungen (1b) senkrecht zu den dehnbaren Kettfäden liegen.

3. Textiler Artikel gemäß einem der Ansprüche 1 oder 2, ***dadurch gekennzeichnet, dass*** der Titer des Silikonfadens (1) oder des silikongetränkten Fadens zwischen ungefähr 1.500 und 2.500 dtex liegt.

4. Textiler Artikel nach einem der Ansprüche 1 oder 2, ***dadurch gekennzeichnet, dass*** der Silikonfaden (1) oder der silikongetränkte Faden einen runden Querschnitt haben.

5. Textiler Artikel nach einem der Ansprüche 1 oder 2, ***dadurch gekennzeichnet, dass*** der Silikonfaden (1) oder der silikongetränkte Faden einen ovalen Querschnitt haben.

6. Textiler Artikel nach einem der Ansprüche 1 oder 2, ***dadurch gekennzeichnet, dass*** der Silikonfaden (1) oder der silikongetränkte Faden durchscheinend sind.

7. Textiler Artikel nach einem der Ansprüche 1 oder 2, ***dadurch gekennzeichnet,* dass** der Silikonfaden (1) oder der silikongetränkte Faden matt sind.

## Claims

1. Textile article in the form of an aerated strip obtained by knitting or weaving, and suitable for constituting the upper part of a stocking or a sock, which article has on at least one side warp float (la) made of silicone yarns (1) or silicone-coated yarns, in combination with a weave suitable for giving said strip an elongation capacity to be stretchable in warp and a weft lock, ***characterized* in that** the title of the silicone yarn (1) or silicone-coated yarn forming the chain floats (la) is between about 800 and 3500 dtex;
and **in that**:
- in the case of a knit, the warp floats (la) are parallel to the warps (2) which incorporate a weave having elongation capacities, the weft being made of rigid yarn (3), not extensible when subjected to manual pulling, under normal temperature and pressure conditions (CNTP); and
- in the case of a weave, warp floats (la) are perpendicular to the weft yarns (3) which are rigid, not extensible when exposed to manual pulling, under normal temperature and pressure conditions (CNTP), and the warp yards having an elongation capability.

2. Textile article in the form of an aerated strip obtained by knitting or weaving, and suitable for forming the upper part of a stocking or a sock which article has on at least one side of weft float (1b) made of silicone yarns (1) or silicone-coated yarns, in combination with a weave suitable for giving said strip an elongation capacity to be stretchable in warp and a weft lock, ***characterized* in that** the title of the silicone yarn (1) or silicone-coated yarn forming the warp floats (la) is between about 800 and 3500 dtex;
and **in that**:
- in the case of a knit, the weft floats (1b) are perpendicular to the warps (2) which incorporate a core with elongation capacities ; and
- in the case of a weave, the weft floats (1b) are perpendicular to the warp yarns having an elongation capacity.

3. Textile article according to any of claims 1 or 2, ***characterized* in that** the title of the silicone yarn (1) or silicone-coated yarn is between 1500 and 2500 dtex.

4. Textile article according to any of claims 1 or 2, ***characterized* in that** the silicone yarn (1) or silicone-coated yarn has a round cross-section.

5. Textile article according to any of claims 1 or 2, ***characterized* in that** the silicone yarn (1) or silicone-coated yarn has an oval cross-section.

6. Textile article according to any of claims 1 or 2, ***characterized* in that** the title of the silicone yarn (1) or silicone-coated yarn is translucent.

7. Textile article according to any of claims 1 or 2, ***characterized* in that** the silicone yarn (1) or silicone-coated yarn is matt.
